(19) Europäisches Patentamt
European Patent Office
Office européen des brevets

(11) Numéro de publication: **0 066 489**
**B1**

(12) # FASCICULE DE BREVET EUROPEEN

(45) Date de publication du fascicule du brevet: **13.11.85**

(51) Int. Cl.⁴: **C 07 D 453/04**, A 61 K 31/49

(21) Numéro de dépôt: **82400867.6**

(22) Date de dépôt: **11.05.82**

(54) **Nouveaux dérivés de la quinidine, procédé de préparation et application en thérapeutique.**

(30) Priorité: **20.05.81 FR 8110024**

(43) Date de publication de la demande:
**08.12.82 Bulletin 82/49**

(45) Mention de la délivrance du brevet:
**13.11.85 Bulletin 85/46**

(84) Etats contractants désignés:
**BE CH DE FR GB IT LI LU NL SE**

(56) Documents cités:
**XENOBIOTICA, vol. 11, no. 2, février 1981, pages 81-87, Londres (GB); C. LIDDLE et al.: "Identification of new urinary metabolites in man of quinine using methane chmeical ionization gas chromatography-mass spectrometry"**
**TETRAHEDRON LETTERS, no. 21, 1976, pages 1757-1760, Oxford (GB); F.I. CARROLL et al.: "Synthesis and stereochemistry of a metabolite resulting from the biotransformation of quinidine in man"**
**L.K. Lowetal: "Drug Biotransformations" - Burger's Medicinal Chemistry, 1980, p. 136**
**H.O. House "Modern Synthetic Reactions", p. 13**

**Le dossier contient des informations techniques présentées postérieurement au dépôt de la demande et ne figurant pas dans le présent fascicule.**

(73) Titulaire: **ETABLISSEMENTS NATIVELLE S.A., 27, rue de la Procession, F-75015 Paris (FR)**

(72) Inventeur: **Jarreau, François-Xavier, 5, rue Louis Hervé, F-78000 Versailles (FR)**
Inventeur: **Koenig, Jean-Jacques, 31, rue du Panorama, F-77670 Vernou la Celle s/Seine (FR)**

(74) Mandataire: **L'Helgoualch, Jean et al, OFFICE PICARD 134 Boulevard de Clichy, F-75018 Paris (FR)**

**EP 0 066 489 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

## Description

La présente invention concerne de nouveaux dérivés de la quinidine, un procédé pour leur préparation ainsi que leur application en thérapeutique.

On sait que la quinidine possède des propriétés thérapeutiques qui permettent son application pour le traitement de diverses arythmies cardiaques, ventriculaires ou supra-ventriculaires. L'administration de la quinidine peut toutefois s'accompagner de certains inconvénients comme par exemple des troubles digestifs, et il est souvent apparu souhaitable de pouvoir disposer de médicaments présentant les propriétés essentielles de la quinidine, avec éventuellement une efficacité plus grande, mais dépourvus de certains effets secondaires gênants.

On connait certains composés dérivés de la quinidine obtenus par salification au moyen d'un agent approprié, tels que l'alginate de quinidine décrit au brevet français 2 115 199 ou un polysaccharide sulfate de quinidine comme décrit au brevet français 2 013 170 ou encore le galacturonate de dihydroquinidine comme décrit au brevet US 3 479 359. Toutefois, si de tels dérivés permettent parfois d'atténuer certains inconvénients de la quinidine, ils ne peuvent en améliorer ou en modifier l'activité, et leur utilisation ne s'avère pas toujours satisfaisante.

La présente invention a pour objet de nouveaux dérivés de la dihydroquinidine exerçant des activités thérapeutiques proches de celles de la quinidine, et plus particulièrement de nouveaux dérivés de la dihydroquinidine hydroxylés en position 3, utilisables en thérapeutique, susceptibles d'être mieux adaptés au traitement des arythmies cardiaques, ainsi qu'un procédé de préparation de ces dérivés.

Les nouveaux composés conformes à la présente invention sont l'hydroxy-3 dihydro-10,11 quinidine, et ses dérivés, représentés par la formule générale (I):

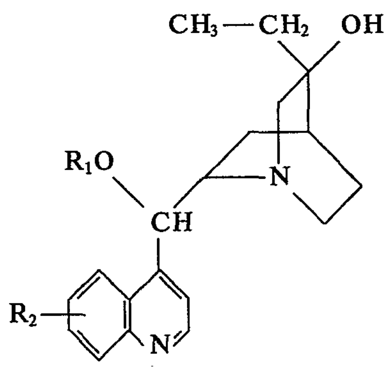

(I)

dans laquelle $R_1$ représente un atome d'hydrogène, un groupe alkyle ou un groupe acyle, et $R_2$ représente un atome d'hydrogène, un groupe hydroxy, un groupe alcoxy, un groupe acyloxy ou un groupe aroyloxy.

Dans la formule générale (I) ci-dessus, $R_1$ peut représenter notamment un atome d'hydrogène ou un groupe alkyle, de préference un groupe alkyle inférieur de 1 à 4 atomes de carbone tel que méthyle, éthyle, isopropyle, etc., ou un groupe acyle tel que formyle, acétyle, butyroyle, etc. $R_2$ peut représenter un atome d'hydrogène, un groupe hydroxy, un groupe alcoxy, et de préference un groupe alcoxy inférieur de 1 à 4 atomes de carbone tel que méthoxy, éthoxy, isopropoxy, n-propoxy, etc., un groupe acyloxy et de préférence un groupe acétoxy ou formyloxy, ou encore un groupe aroyloxy tel qu'un groupe benzoyloxy. Le substituant représenté par $R_2$ peut être fixé sur diverses positions du noyau quinolinyle, et occupe de préférence la position 6'.

L'invention concerne de préférence les composés de formule générale (I) dans laquelle $R_1$ est un atome d'hydrogène, ou un groupe acétyle, et $R_2$ est un atome d'hydrogène ou un groupe alcoxy inférieur, hydroxy ou acétoxy, en position 6'.

L'invention concerne notamment les isomères des dérivés de formule (I), et en particulier l'hydroxy-3S dihydroquinidine, l'hydroxy-3R dihydroquinidine, et le mélange d'épimères en position 3, dont l'existence est liée à la présence d'un carbone asymétrique en position 3. Ces isomères sont obtenus à partir de l'hydroxy-3S quinidine, de l'hydroxy-3R quinidine, et du mélange d'épimères en position 3, respectivement, comme décrit plus en détail ci-après.

Conformément à la présente invention les dérivés de la quinidine de formule générale (I) ci-dessus peuvent être préparés à partir des dérivés de formule générale (II):

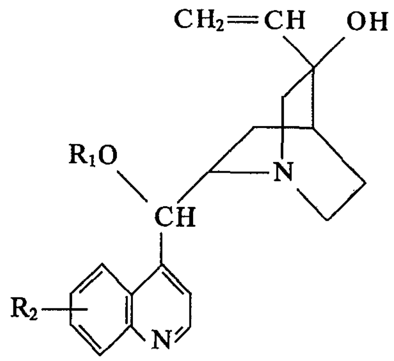

(II)

dans laquelle $R_1$ est un atome d'hydrogène ou un groupe acétyle et $R_2$ est un atome d'hydrogène ou un groupe méthoxy, en effectuant une réaction d'hydrogénation, suivie le cas échéant d'une acylation ou d'une desméthylation.

La réaction d'acylation effectué le cas échéant après la réaction d'hydrogénation permet d'obtenir les produits de formule (I) où $R_1$ est un groupe acyle à partir des dérivés de formule (II) où le groupe $R_1$ correspondant est un atome d'hydrogène; on peut de même effectuer une alkylation pour obtenir les produits où $R_1$ est un groupe alkyle. La réaction de desméthylation est effectuée le cas échéant pour transformer le groupe méthoxy représenté par $R_2$ dans la formule (II) en un groupe hydroxy; en effectuant ensuite une alkylation ou une acylation, on obtient les produits de formule (I) où $R_2$ est un groupe alcoxy ou acyloxy (ou aroyloxy) respectivement. Toutes ces réactions sont effectuées suivant les techniques usuelles.

Les dérivés de l'hydroxy-3 quinidine de formule (II) sont des composés connus qui peuvent être préparés par des méthodes usuelles. Par exemple l'hydroxy-3 quinidine représentée par la formule (II) dans laquelle $R_1$ est un atome d'hydrogène et $R_2$ est un groupe méthoxy, est un métabolite connu de la quinidine qui peut être préparé par la méthode de F. I. Carroll et al. Tetrahedron n° 21, p. 1757—1760 (1976). Cette méthode peut également être appliquée pour la préparation des autres composés de formule générale (II).

Des dérivés de l'hydroxy-3 dihydroquinidine sont décrits dans »Xenobiotica«, 1981, Vol. II, N° 2, p. 81—87.

La réaction d'hydrogénation permettant d'obtenir les dérivés d'hydroxy-3 dihydro-10,11 quinidine à partir des composés de formule générale (II) peut s'effectuer par les techniques usuelles. La réaction d'hydrogénation catalytique peut s'effectuer au moyen d'un catalyseur à base de métal noble tel que le platine, le palladium ou le rhodium, ou d'un catalyseur au nickel Raney. Le palladium ou le rhodium employé comme catalyseur est déposé sur un support tel que le carbone ou l'alumine.

On peut par exemple effectuer une hydrogénation catalytique en dissolvant le composé de formule (II) dans un solvant organique approprié sous atmosphère d'hydrogène en présence d'un catalyseur palladium/carbone.

Comme solvant on peut utiliser par exemple un alcool tel que le méthanol, l'éthanol ou l'isopropanol. La réaction peut s'effectuer à température ambiante, sous pression normale.

L'invention concerne également les sels des dérivés de la quinidine représentés par la formule générale (I) ci-dessus, et en particulier les sels pharmaceutiquement acceptables, obtenus en faisant agir un acide minéral ou organique sur le dérivé de quinidine comme base. Ces sels peuvent être obtenus par les méthodes usuelles de la technique, en faisant réagir le dérivé de quinidine et l'acide, en proportions sensiblement stoechiométriques, dans un solvant compatible. L'acide peut être par exemple l'acide chlorhydrique, l'acide lactique, l'acide oxalique, l'acide phosphorique, l'acide bromhydrique, l'acide formique, l'acide sulfurique, l'acide tartrique, l'acide maléique, etc.

Il est intéressant d'observer que l'hydroxy-3 quinidine, métabolite connu de la quinidine, présente certaines propriétés pharmacologiques comparables à celles de la quinidine, qui n'ont cependant pas justifié des études cliniques plus approfondies, et bien qu'il soit connu, ce composé n'est pas appliqué en thérapeutique. Il peut paraitre surprenant dans ces conditions que l'hydroxy-3 dihydro-10,11 quinidine, composé correspondant dont la double liaison en 10—11 a été réduite, possède une activité pharmacologique non seulement comparable à celle de la quinidine, mais même supérieure pour certaines propriétés.

Les propriétés pharmacologiques et les applications thérapeutiques des dérivés de l'hydroxy-3 dihydro-10,11 quinidine conformes à la présente invention, ainsi que des exemples non limitatifs de préparation de composés préférés, sont décrits plus en détail ci-après.

## Exemple 1

### Hydroxy-3S dihydro-10,11 quinidine

On dissout 0,7 g d'hydroxy-3S quinidine dans 10 ml de méthanol. On ajoute 65 mg de catalyseur au palladium sur charbon et on place le milieu réactionnel sous atmosphère d'hydrogène pendant 10 mn.

La réaction étant terminée, on filtre le catalyseur. Le filtrat est évaporé à sec puis le résidu est recristallisé dans de l'acétate d'éthyle.

On obtient ainsi l'hydroxy-3S dihydro-10,11 quinidine avec un rendement de 90%.

Point de fusion F = 210° C (acétate d'éthyle)
Spectre IR (Nujol) $\nu$ = 3600 à 2200 (pics à 3510 et 3320), 1620, 1590, 1565 et 1510 $cm^{-1}$
Spectre de RMN ($CD_3OD$) $\delta$ = 0,95 (t, 3H); 1,1 à 3,9 (12H); 3,9 (s, 3H); 5,6 (d, 1H); 7,2 à 8,0 et 8,6 (5H) ppm.

## Exemple 2

### Hydroxy-3R dihydro-10,11 quinidine

On procède comme dans l'exemple 1 en remplaçant l'hydroxy-3S quinidine par de l'hydroxy-3R quinidine. On obtient alors l'hydroxy-3R dihydro-10,11 quinidine avec un rendement supérieur à 90%.

Point de fusion F = 189° C (acétate d'éthyle)
Spectre IR (Nujol) $\nu$ = 3600 à 2400 (pics à 3440, 3100 et 2740), 1620, 1585, 1570 et 1510 $cm^{-1}$.
Spectre de RMN ($CDCl_3$) $\delta$ = 0,9 (t, 3H); 1,2 à 3,5 (13H); 3,8 (s, 3H); 5,0 (1H, mobile); 5,56 (s, 1H); 7,0 à 8,4 (5H) ppm.

## Exemple 3

### Hydroxy-3S O-acétyl dihydro-10,11 quinidine

On dissout 0,6 g d'hydroxy-3S dihydro-10,11 quinidine obtenue comme indiqué dans l'exemple 1 dans 10 ml de chlorure de méthylène et dans cette solution on verse goutte à goutte 0,3 g d'anhydride acétique. Le produit formé est recueilli et purifié suivant les techniques usuelles.

On obtient ainsi l'hydroxy-3S O-acétyl dihydro-10,11 quinidine avec un rendement de 95%.

Point de fusion: F = 130—132° C (acétate d'éthyle)
Spectre IR (Nujol) $\nu$ = 3500—2500 (maximum à 3060), 1735, 1615, 1585, 1565, 1505 $cm^{-1}$.

Les expérimentations effectuées sur les dérivés d'hydroxy-3 dihydroquinidine ont mis en évidence d'intéressantes propriétés pharmacologiques montrant que les dérivés conformes à l'invention peuvent être utilisés en thérapeutique vétérinaire et humaine.

### Propriétés toxicologiques

La valeur de la dose léthale DL 50 a été mesurée suivant la méthode de Lichfield et Wilcoxon (J. Pharmacol. 1949, 96, 99—113) sur la souris et le rat (10 animaux: 5 mâles et 5 femelles par dose).

Les valeurs relevées pour la DL 50 sont de l'ordre de 90 à 150 mg/kg par injection intraveineuse (IV), 200 à 400 mg/kg par injection intrapéritonéale (IP) et 850 à 1100 mg/kg par administration per os chez la souris. La valeur de la DL 50 chez le rat est de l'ordre de 600 à 700 mg/kg par administration per os.

Par exemple, les résultats obtenus avec l'hydroxy-3S dihydro-10,11 quinidine sous forme de chlorhydrate sont exprimés au tableau 1 suivant.

Tableau 1

| | souris | | | | | | rat | |
|---|---|---|---|---|---|---|---|---|
| | sexe mâle | | | femelle | | | mâle | femelle |
| | administration IV | IP | per os | IV | IP | per os | per os | per os |
| DL 50 (mg/kg) | 120 | 280 | 975 | 105 | 180 | 900 | 640 | 640 |

A titre de comparaison, les valeurs moyennes de la DL 50 pour la quinidine par administration intrapéritonéale chez la souris (mâle et femelle) sont de 225—235 mg/kg. Ces valeurs montrent que le toxicité des dérivés de la quinidine conformes à l'invention n'est pas augmentée par rapport à la quinidine, mais semble au contraire atténuée.

## Propriétés pharmacologiques

Les effets hémodynamiques provoqués par des doses cumulatives d'hydroxy-3S dihydro-10,11 quinidine chez le chien ont été mesurés. Les résultats sont regroupés au tableau 2 ci-après.

Les paramètres ont été enregistrés à l'aide de:

- cathéters reliés à des capteurs de pression (pression artérielle, pression ventriculaire gauche et sa dérivée première dp/dt);
- débitmètre électromagnétique sur l'aorte (débit aortique);
- électrocardiographe;
- jauge de contrainte placée sur la myocarde ventriculaire gauche (force de contraction myocardique).

Les dérivés sont injectés par voie intraveineuse, une dose toutes les 30 minutes environ et leurs effets sont mesurés 20 minutes après la fin de l'injection (durée de l'injection: 2 minutes).

Les résultats regroupés dans ce tableau, exprimés en variation par rapport à la valeur initiale, 20 mn après injection, montrent que le dérivé étudié chez le chien entraîne les modifications suivantes:

- la pression artérielle systolique diminue progressivement avec la dose, les effets ne devenant importants que pour une dose cumulative supérieure à 10 mg/kg;
- la fréquence cardiaque n'est pas modifiée de manière significative avant la dose cumulative de 20 mg/kg;
- de même, le débit cardiaque ne subit aucune modification significative avant la dose cumulative de 20 mg/kg;
- on n'observe aucune modification de la force de contraction myocardique.

Un peut en conclure que le tolérance cardiovasculaire chez le chien est satisfaisante, puisque les effets sont limités à une chute modérée de la pression artérielle, sans modification de la force contractile myocardique et sans diminution importante du débit cardiaque, pur des doses cumulatives inférieures à 20 mg/kg.

Le tableau 3 ci-après regroupe les données électrophysiologiques relatives à l'administration de l'hydroxy-3S dihydroquinidine, par comparaison avec la quinidine.

L'étude est réalisée chez le chien anesthésié au pentobarbital, à thorax fermé, à l'aide de catheters-électrodes bipolaires introduites dans les cavités cardiaques par voies veineuse et artérielle transcutanée.

On peut ainsi mesurer à l'aide d'un stimulateur programmable, l'automaticité sinusale, les temps de conduction intracardiaque, et les périodes réfractaires cardiaques effectives et fonctionnelles.

Les résultats exposés au tableau 3 exprimant les pourcentages de variations des différents paramètres, montrent que le composé conforme à la présente invention,

- ralentit la fréquence cardiaque comme la quinidine;
- modifie peu la conduction entre les oreillettes et le tronc du faisceau de His, alors qu'il exerce des effets supérieurs à ceux de la quinidine sur la conduction dans le système His-Purkinje (des effets similaires sont observés en ce qui concerne la conduction intraventriculaire);
- exerce, pour les périodes réfractaires cardiaques, des effets équivalents à ceux de la quinidine à l'étage ventriculaire et nodal, et supérieurs à ceux de la quinidine au niveau des oreillettes.

Tableau 2

| | valeurs initiales | Dose cumulative (mg/kg I.V.) 0,5 | +0,5 | +4 | +5 | +10 |
|---|---|---|---|---|---|---|
| Pression artérielle systolique (mm Hg) | 130±6 | −3±3 | −11±4 | −20±5 | −23±7 | −36±13 |
| Fréquence cardiaque (bat./mn.) | 140±11 | 0±0 | −2±6 | −5±2 | −3±16 | −15±13 |

(suite de tableau 2)

| | valeurs initiales | Dose cumulative (mg/kg I.V.) 0,5 | +0,5 | +4 | +5 | +10 |
|---|---|---|---|---|---|---|
| Débit cardiaque (ml/mn.) | 2800±246 | 0±0 | −50±50 | −90±143 | −316±412 | −775±498 |
| Force des contractions du myocarde (g) | 33±3 | −1±1 | −3±3 | 0±1 | 2±1 | 8±1 |
| Résistance périphérique totale | 3119±415 | −111±111 | −382±180 | −457±277 | −376±228 | −91±283 |

Tableau 3

| | Doses (mg/kg) 1 | 5 | 10 |
|---|---|---|---|
| Cycle Cardiaque | | | |
| Invention | + 4,5% | + 5,1% | +14,–% |
| Quinidine | + 2,7 | + 5,1 | + 9,2 |
| SH*) | | | |
| Invention | + 1,7 | + 3,8 | +15 |
| Quinidine | + 3,5 | + 7,2 | +10,5 |
| HV*) | | | |
| Invention | +10,8 | +20,9 | +32,8 |
| Quinidine | 0 | + 8,7 | +17,7 |
| QRS*) | | | |
| Invention | +14,4 | +14,4 | +22 |
| Quinidine | 0 | 0 | 0 |
| PREA*) | | | |
| Invention | + 8 | +16 | +23,7 |
| Quinidine | + 2 | + 3,5 | +14,4 |
| PRFN*) | | | |
| Invention | 0 | + 5,5 | +12,6 |
| Quinidine | + 3 | + 5 | + 9 |
| PREV*) | | | |
| Invention | − 1,5 | + 2,8 | + 6,5 |
| Quinidine | + 3,3 | + 6,5 | + 8,5 |
| $QT_c$*) | | | |
| Invention | + 2,8 | + 7 | + 6 |
| Quinidine | + 1,8 | 0 | + 1,5 |

*) SH: Conduction auriculo-Hissienne
HV: Conductions Hiss-Purkinge
QRS: Conduction, intra-ventriculaire
PREA: Période réfractaire effective auriculaire
PRFN: Période réfractaire fonctionelle nodale
PREV: Période réfractaire effective ventriculaire
$QT_c$: Temps de repolarisation corrigé.

L'activité antiarythmique a été observée chez la souris au moyen du test de Lawson suivant la méthode décrite par J. W. Lawson, J. Pharmacol. Exp. Therp. 1968, 160, 22—31 et Cr. Narcisse et al. Ann. Pharma. Fr. 1979, 37, 325—330, chez le rat par le test de l'aconitine selon S. Witchitz et al. Cœur Méd. Int. 1971, X(2), 281—286 et sur le chien par le test de Harris décrit dans Circulation, 1950, 1, 1318. L'hydroxy-3S dihydro-10,11 quinidine a été utilisée pour chacun de ces trois tests.

Les résultats sont regroupés au tableau 4 ci-après. Dans les trois cas, l'activité a été comparée à celle de la quinidine qui constitue le produit de référence.

Le test de Lawson permet d'étudier le pouvoir antifibrillatoire cardiaque des dérivés. Il montre que l'efficacité antifibrillatoire du dérivé conforme à l'invention est comparable à celle de la quinidine. La dose efficace 50 (ED 50) indiquée au tableau 4 est la dose qui protège la moitié des souris contre la fibrillation ventriculaire anoxique.

Le test à l'aconitine montre que le dérivé suivant l'invention exerce un effet protecteur contre les arythmies supérieur à celui de la quinidine puisqu'il allonge plus que la quinidine le délai d'apparition des extrasystoles ventriculaires (ESV) de la tachycardie ventriculaire (TV) et de la fibrillation ventriculaire (FV).

Les résultats du test de Harris font apparaitre que la diminution des arythmies ventriculaires, à la dose de 6 mg/kg, est plus importante avec le composé suivant l'invention qu'avec la quinidine. On constate en outre que l'efficacité des produits est comparable pour des doses supérieures.

Tableau 4

| Test | Espece animale | Voie administr. | Dose | | Invention | | | Quinidine | |
|---|---|---|---|---|---|---|---|---|---|
| Lawson | Souris | I. P. | — | ED 50: | 72,5 | 6,94 mg/kg | | 54 | 6,58 mg/kg |
| Aconitine | Rat | I. V. | 10 mg/kg | | Délai apparition | | | Délai apparition | |
| | | | | Trouble | mn | % | | % | mn |
| | | | | ESV (98)*) | 208 | +112 | | + 53 | 150 |
| | | | | TV (112) | 253 | +125 | | + 52 | 171 |
| | | | | FV (228) | 504 | +121 | | +107 | 474 |
| Harris | Chien | I. V. | | période observ. | % Protection Exp. 1 | Exp. 2 | Moyen | Période observ. | % Protection |
| | | | 6 mg/kg | 1 h | 65 | 39 | 52 | 1 h | 21 |
| | | | +10 mg/kg | 1 h | 72 | 42 | 57 | +5 mg 1 h | 55 |
| | | | | 2 h | 97 | 41 | 69 | 2 h | 52 |

*) Délai d'apparition contrôle

Ces résultats montrent que les dérivés de quinidine suivant l'invention présentent des propriétés antiarythmiques voisines de celles de la quinidine, sur le plan qualitatif. Sur le plan quantitatif, on peut noter en particulier que l'hydroxy-3S dihydro-10,11 quinidine est nettement plus active que la quinidine sur le test à l'aconitine.

De plus, il faut observer que l'activité électrophysiologique au niveau de la conduction His-ventricule, constitue un avantage par rapport à la quinidine.

Ces propriétés montrent que les dérivés de la quinidine conformes à la présente invention sont utilisables en thérapeutique humaine et vétérinaire dans les mêmes applications que la quinidine, et en particulier dans le traitement de diverses formes d'arythmies cardiaques tant supraventriculaires que ventriculaires.

Les dérivés de quinidine de formule générale (I) et leurs sels pharmaceutiquement acceptables peuvént être administrés sous les formes usuelles, le principe actif étant dilué dans un support pharmaceutiquement acceptable convenablement choisi, par exemple sous forme de comprimé, gélule, dragée, suppositoire, soluté injectable ou sirop.

A titre d'exemple, les comprimés peuvent être préparés en mélangeant le dérivé de quinidine de formule générale (I) ou un de ses sels, avec un ou plusieurs diluants solides tels que le lactose, le mannitol, l'amidon, la polyvinylpyrrolidone, le stéarate de magnésium, le talc, etc. Le cas échéant, les comprimés peuvent comporter plusieurs couches superposées autour d'un noyau, suivant les techniques usuelles, pour assurer une libération progressive ou un effet retardé du principe actif. L'enrobage peut par exemple être constitué d'une ou plusieurs couches d'acétate de polyvinyle, de carboxyméthylcellulose ou d'acétophtalate de cellulose.

On peut également administrer le dérivé suivant l'invention sous forme d'un sirop ou d'un soluté buvable obtenu en dissolvant le dérivé de formule (I) ou un de ses sels pharmaceutiquement acceptables, dans de l'eau ou du glycérol, par exemple, et en ajoutant le cas échéant un additif usuel tel qu'un édulcorant et un antioxydant.

Des solutions injectables peuvent être préparées suivant les techniques bien connues et sont constituées par exemple par un soluté contenant un dérivé de formule (I) ou un de ses sels pharmaceutiquement acceptables, dissous dans de l'eau bidistillée, une solution hydroalcoolique, du propylèneglycol, etc., ou un mélange de ces solvants. Le cas échéant, un additif approprié tel qu'un conservateur peut être ajouté.

La posologie peut varier suivant la nature de l'affection traitée, et le sujet concerné. Les doses administrées journellement sont généralement comparables à celles des traitements quinidiniques, mais peuvent être ajustées par le praticien en fonction des circonstances.

**Revendications**

1. Dérivés de quinidine de formule générale (I):

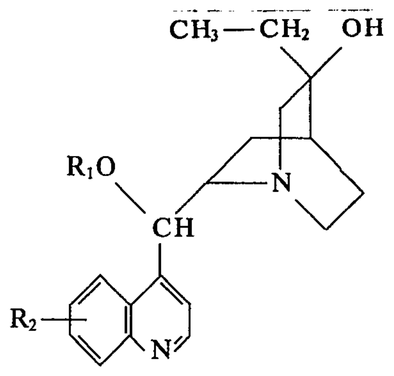

(I)

dans laquelle

$R_1$ représente un atome d'hydrogène, un groupe alkyle inférieur de 1 à 4 atomes de carbone, ou acyle comportant de 1 à 4 atomes de carbone, et

$R_2$ représente un atome d'hydrogène, un groupe hydroxy, un groupe alcoxy inférieur de 1 à 4 atomes de carbone, un groupe acétoxy ou formyloxy, ou un groupe benzoyloxy,

ainsi que leurs sels d'addition d'acides.

2. Dérivés selon la revendication 1, caractérisés en ce que $R_1$ est un atome d'hydrogène, un groupe alkyle inférieur de 1 à 4 atomes de carbone, ou un groupe acyle comportant de 1 à 4 atomes de carbone, et $R_2$ est un atome d'hydrogène, un groupe hydroxy, un groupe alcoxy inférieur de 1 à 4 atomes de carbone, ou un groupe acétoxy.

3. Dérivés selon la revendication 1, caractérisés en ce que $R_1$ est un atome d'hydrogène ou un

groupe méthyle ou acétyle, et $R_2$ est un atome d'hydrogène ou un groupe hydroxy, acétoxy, méthoxy, éthoxy ou propoxy, en position 6'.

4. Dérivé selon l'une quelconque des revendications 1 à 3, caractérisé en ce qu'il est choisi parmi l'hydroxy-3S dihydro-10,11 quinidine, l'hydroxy-3R dihydro-10,11 quinidine, l'hydroxy-3R O-acétyl dihydro-10,11 quinidine, et l'hydroxy-3S O-acétyl dihydro-10,11 quinidine.

5. Composition pharmaceutique, caractérisée en ce qu'elle contient un dérivé selon l'une quelconque des revendications 1 à 4, ou un sel pharmaceutiquement acceptable, dilué le cas échéant dans un support approprié.

6. Procédé de préparation des dérivés selon la revendication 1, caractérisé en ce qu'on effectue une hydrogénation des composés de formule générale (II):

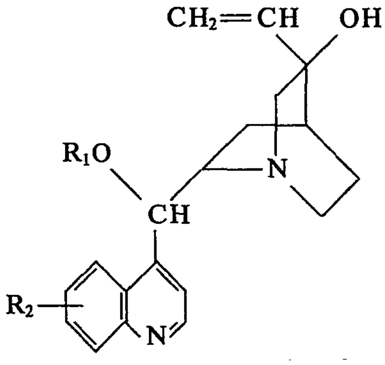

(II)

dans laquelle

$R_1$ est un atome d'hydrogène ou un groupe acétyle, et
$R_2$ est un atome d'hydrogène ou un groupe méthoxy,

suivie le cas échéant d'une acétylation ou d'une desméthylation.

7. Procédé selon la revendication 6, caractérisé en ce que la réaction d'hydrogénation est une hydrogénation catalytique aumoyen d'un catalyseur à base de métal noble ou de nickel Raney.

8. Procédé selon la revendication 7, caractérisé en ce que le catalyseur est du palladium sur carbone.

9. Procédé selon la revendication 6, caractérisé en ce qu'on dissout le composé de formule (II) dans un solvant organique et on opère sous atmosphère d'hydrogène en présence d'un catalyseur palladium/carbone.

**Patentansprüche**

1. Chinidinderivate der allgemeinen Formel (I):

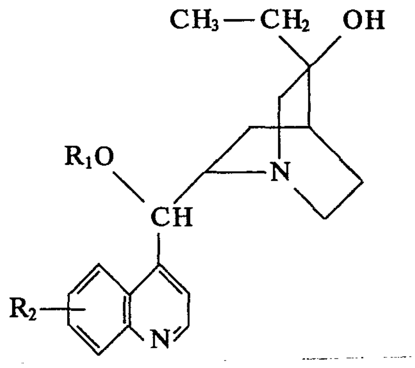

(I)

worin bedeuten:

$R_1$ ein Wasserstoffatom, eine Alkylgruppe mit 1 bis 4 Kohlenstoffatomen oder Acylgruppe mit 1 bis 4 Kohlenstoffatomen und
$R_2$ ein Wasserstoffatom, eine Hydroxygruppe, Alkoxygruppe mit 1 bis 4 Kohlenstoffatomen, Acetoxy- oder Formyloxygruppe oder eine Benzoyloxygruppe

sowie deren Säureadditionssalze.

2. Derivate nach Anspruch 1, dadurch gekennzeichnet, daß $R_1$ ein Wasserstoffatom, eine Alkylgruppe mit 1 bis 4 Kohlenstoffatomen oder eine Acylgruppe mit 1 bis 4 Kohlenstoffatomen und $R_2$ ein

Wasserstoffatom, eine Hydroxygruppe, Alkoxygruppe mit 1 bis 4 Kohlenstoffatomen oder eine Acetoxygruppe bedeuten.

3. Derivate nach Anspruch 1, dadurch gekennzeichnet, daß $R_1$ ein Wasserstoffatom oder eine Methyl- oder Acetylgruppe und $R_2$ ein Wasserstoffatom, eine Hydroxy-, Acetoxy-, Methoxy-, Ethoxy- oder Propoxygruppe in 6'-Stellung bedeuten.

4. Derivat nach einem der Ansprüche 1 bis 3, dadurch gekennzeichnet, daß es gewählt wird aus Hydroxy-3S-dihydro-10,11-chinidin, Hydroxy-3R-dihydro-10,11-chinidin, Hydroxy-3R-O-acetyl-dihydro-10,11-chinidin und Hydroxy-3S-O-acetyl-dihydro-10,11-chinidin.

5. Arzneimittel, dadurch gekennzeichnet, daß es ein Derivat nach einem der Ansprüche 1 bis 4 oder ein pharmazeutisch annehmbares Salz hiervon, gegebenenfalls in Verdünnung mit einer geeigneten Trägersubstanz, enthält.

6. Verfahren zur Herstellung der Derivate nach Anspruch 1, dadurch gekennzeichnet, daß man Verbindungen der allgemeinen Formel (II):

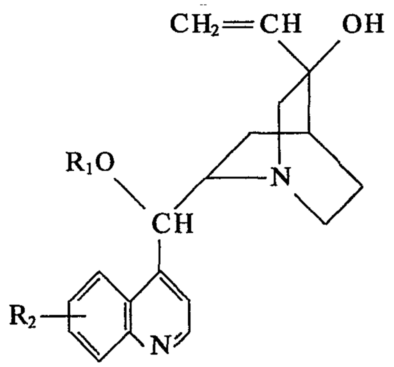

(II)

worin

$R_1$ ein Wasserstoffatom oder eine Acetylgruppe und
$R_2$ ein Wasserstoffatom oder eine Methoxygruppe

bedeuten, hydriert und gegebenenfalls daran anschließend eine Acetylierung oder eine Entmethylierung durchführt.

7. Verfahren nach Anspruch 6, dadurch gekennzeichnet, daß es sich bei der Hydrierungsreaktion um eine katalytische Hydrierung mittels eines Katalysators auf Grundlage eines Edelmetalls oder von Raney-Nickel handelt.

8. Verfahren nach Anspruch 7, dadurch gekennzeichnet, daß es sich bei dem Katalysator um Palladium-auf-Kohlenstoff handelt.

9. Verfahren nach Anspruch 6, dadurch gekennzeichnet, daß man die Verbindung der Formel (II) in einem organischen Lösungsmittel löst und unter Wasserstoffatmophäre in Gegenwart eines Palladium-Kohlenstoff-Katalysators arbeitet.

## Claims

1. Quinidine derivatives of general formula (I):

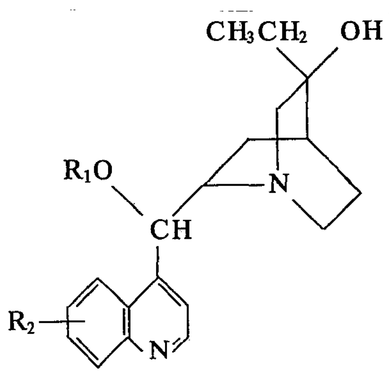

(I)

wherein

$R_1$ represents a hydrogen atom, a lower alkyl group with 1 to 4 carbon atoms or an acyl group comprising 1 to 4 carbon atoms, and

$R_2$ represents a hydrogen atom, a hydroxy group, a lower alkoxy group with 1 to 4 carbon atoms, an acetoxy or formyloxy group or a benzoyloxy group,

and the acid addition salts thereof.

2. The derivatives of claim 1, characterized in that $R_1$ is a hydrogen atom, a lower alkyl group with 1 to 4 carbon atoms, or an acyl group comprising 1 to 4 carbon atoms and $R_2$ is a hydrogen atom, a hydroxy group, a lower alkoxy group with 1 to 4 carbon atoms, or an acetoxy group.

3. The derivatives of claim 1, characterized in that $R_1$ is a hydrogen atom, a methyl group or an acetyl group, and $R_2$ is a hydrogen atom, a hydroxy group, an acetoxy group, a methoxy group, an ethoxy group or a propoxy group, at the 6′-position.

4. The derivative of any of claims 1 to 3, characterized in that it is selected from the group consisting of 3S-hydroxy-10,11-dihydroquinidine, 3R-hydroxy-10,11-dihydroquinidine, 3R-hydroxy-O-acetyl-10,11-dihydroquinidine, and 3S-hydroxy-O-acetyl-10,11-dihydroquinidine.

5. A pharmaceutical composition characterized in that it comprises a derivative of any of claims 1 to 4, or a pharmaceutically acceptable salt thereof, possibly diluted in an acceptable carrier.

6. A process for preparation of the derivatives of claim 1, characterized in that it comprises hydrogenating compounds of general formula (II):

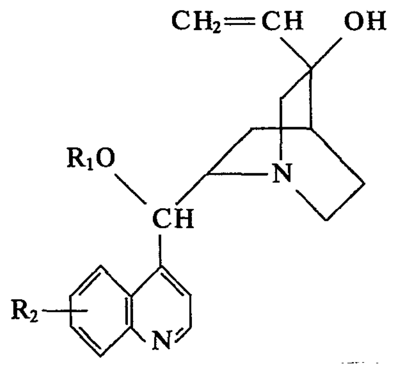

(II)

wherein

$R_1$ is a hydrogen atom or an acetyl group, and
$R_2$ is a hydrogen atom or a methoxy group,

followed as necessary by acetylating or demethylating the product obtained.

7. The process of claim 6, characterized in that the hydrogenating is a catalytic hydrogenating using a noble metal or Raney nickel-based catalyst.

8. The process of claim 7, characterized in that the catalyst is palladium-on-carbon.

9. The process of claim 6, characterized in that the compound of formula (II) is dissolved in an organic solvent, and the reaction is carried out under a hydrogen atmosphere in the presence of a palladium/carbon catalyst.